# EUROPEAN PATENT APPLICATION

(11) **EP 4 385 447 A1**
(43) Date of publication of application: **19.06.2024**
(21) Application number: 22306860.2
(22) Date of filing: 13.12.2022
(51) Int. Cl.: A61B 34/20, A61B 34/30, A61B 34/00, A61B 90/50, A61B 17/00, A61B 90/00

(54) **END-EFFECTOR FOR A ROBOTIC ARM ADAPTED TO MINIMIZE THE IMPACT OF MAGNETIC DISTURBANCES WHEN USED WITH ELECTROMAGNETIC LOCALIZATION SYSTEMS**

(71) Applicant: Spinem Robotics, 38400 Saint-Martin-D'Hères (FR)
(72) Inventor: MORVAN, Stéphane, 38400 SAINT-MARTIN-D'HÈRES (FR)
(74) Representative: Regimbeau

(57) **Abstract**

The present invention concerns an end-effector (210) for a robotic arm (102) adapted to be used with a surgical tool to treat a region of interest of an anatomical structure, the end-effector (210) comprising:
a. a mounting part (211) adapted to be attached to a flange (105) of the robotic arm (102),
b. a hollow part (213) extending mainly along a first axis (A1), the hollow part (213) being rigidly attached to the mounting part (211),
c. a first electromagnetic transducer (301) attached to the hollow part (213), the first electromagnetic transducer (301) being mobile around the first axis (A1), the first axis (A1) and a second axis (A2) passing through the first electromagnetic transducer (301) and crossing the first axis (A1) defining a reference plane (P1),
d. a tubular part (214) received in the hollow part (213), the tubular part (214) being in a fixed position with respect to the first electromagnetic transducer (301),
e. a guiding part (400) configured to be received in the tubular part (214) and configured to receive the surgical tool, the guiding part (400) being equipped with a second electromagnetic transducer (302),
wherein the tubular part (214) comprises a first cooperative surface (244) adapted to cooperate, in a keyed connection, with a second cooperative surface (403) formed on the guiding part (400) and wherein the keyed connection is adapted to ensure that at least part of the second electromagnetic transducer (302) lies in the reference plane (P1).

## Description

The present invention pertains to the domain of robotically aided orthopedic surgeries, and especially to an end-effector of a robotic arm particularly adapted to be used with electromagnetic localization systems.

During the last few decades, the field of computer-assisted systems has significantly grown, and especially computer-assisted surgery systems which are adapted to be used in computer-assisted medical intervention (CAMI), as referred to in the publication of S. Lavallée & P. Cinquin: "Computer assisted medical interventions" In K.H. Hohne, editor, NATO ARW, 30 Imaging in Medicine, Vol F60, 301-312, Berlin, June 1990. Springer-Verlag. Such systems aim to help surgeons in performing safer surgical treatments while also improving the accuracy, precision and reproducibility of said treatments. The use of those computer-assisted surgery systems also aims at improving the precision of surgical actions, also lowering invasiveness during those treatments. Those computer-assisted systems are, most of the time, used in orthopedic surgeries.

Robotic aid to clinicians for the execution of optimal surgery was introduced in the early 1980's in neurosurgery application. Since the 1990's, several assistive technologies combining part or all of 2D/3D imaging, navigation and robotics were developed with the primary focus of improving accuracy of surgical procedures, in view of improving clinical and functional outcomes for the patients.

In most of currently used systems, the system comprises a robotic arm holding a surgical tool. The robotic arm can be automated, or it can be displaced by a surgeon, directly or through an input device. Those systems are often associated with navigation systems adapted to determine the current poses of different objects with respect to one another. Those objects most of the time include, at least, one or several anatomical structures, the surgical tool and the robotic arm.

In currently used systems, the navigation is most often realized thanks to optical localization systems. Such optical navigation systems comprise one or several optical trackers adapted to be detected by one or more stereo cameras which are then adapted to process the information to determine the pose of the objects to which the optical trackers are attached. There are at least three major drawbacks to these optical localization systems.

First, these systems are particularly sensitive to line-of-sight issues. Indeed, if someone or something is positioned between one of the optical trackers and the camera(s), said camera(s) can no longer detect the concerned trackers and, consequently, can no longer determine the pose of the object to which said optical tracker is attached. Operating room are generally busy spaces wherein such situations can easily occur. Furthermore, some blood projections or tissue projections can stain the optical trackers, the formed stain thus preventing the camera(s) to unambiguously determine the pose of the concerned trackers.

A second drawback of those optical localization systems lies on the size of the optical trackers which are made, for most of them, of reflective spheres attached to rigid rods, themselves attached to the objects to be tracked. Those optical trackers thus increase the bulkiness of the general system, which can make the surgeon's work more difficult as they are attached on, or close to, the anatomical structure(s) to be treated.

Recently, electromagnetic localization systems have been developed to avoid the drawbacks of optical systems. Those electromagnetic localization systems comprise one or several electromagnetic emitter(s) adapted to emit at least one electromagnetic field, and one or several electromagnetic receivers adapted to receive and measure these emitted electromagnetic field(s). The electromagnetic field measurements are then used to determine the pose of the electromagnetic receiver(s) with respect to the electromagnetic emitter(s). Consequently, the pose of the objects to which are attached said electromagnetic transducers - either emitters or receivers - can then be determined. Using these electromagnetic localization systems solves all the drawbacks of the optical localization systems described above. First, there is no need of an unobstructed, free space between the receivers and the emitters, as there are no line-of-sight issues. Second, the electromagnetic transducers are made of small coils, thus resulting in smaller trackers than the optical ones and the global system thus being less bulky than optical localization systems.

One drawback of those electromagnetic localization systems is that they are very sensitive to the presence of metallic material in their surroundings. For instance, ferrous metals, have high magnetic permeability, and as such represent a preferred pathway for the emitted magnetic field(s), resulting in magnetic disturbances. Metallic materials are indeed known to generate magnetic disturbances that distort the emitted electromagnetic field, thus making the measurements unreliable to determine the poses of the tracked objects.

Thus, there remain a need to improve the existing systems, and especially for the navigation part. The invention disclosed herein describes a new kind of end-effector adapted to be used with electromagnetic localization system by ensuring that the electromagnetic receivers are as close as possible to the electromagnetic emitter, thus canceling, or at least significantly reducing, the potential effect of the presence of disturbing materials in the system surroundings.

An object of the present invention thus concerns an end-effector for a robotic arm adapted to be used with a surgical tool to treat a region of interest of an anatomical structure, the end-effector comprising:
a mounting part adapted to be attached to a flange of the robotic arm,
a hollow part extending mainly along a first axis, the hollow part being rigidly attached to the mounting part,
a first electromagnetic transducer attached to the hollow part, the first electromagnetic transducer being mobile around the first axis, the first axis and a second axis passing through the first electromagnetic transducer and crossing the first axis, defining a reference plane,
a tubular part received in the hollow part, the tubular part being in a fixed position with respect to the first electromagnetic transducer,
a guiding part configured to be received in the tubular part and configured to receive the surgical tool, the guiding part being equipped with a second electromagnetic transducer,
wherein the tubular part comprises a first cooperative surface adapted to cooperate, in a keyed connection, with a second cooperative surface formed on the guiding part and wherein the keyed connection is adapted to ensure that at least part of the second electromagnetic transducer lies in the reference plane.

According to an aspect of the invention, the tubular part extends, mainly, along a third axis, and the first cooperative surface is formed so that a translation of the second cooperative surface along the third axis and against the first cooperative surface is transformed into a rotation of the second cooperative surface with respect to the first cooperative surface, around the third axis.

According to one embodiment of the invention, the first cooperative surface comprises at least one chamfer. According to another embodiment of the invention, the first cooperative surface is formed as a helical surface. The helical surface can advantageously be formed on an angular sector of an insertion end of the tubular part greater than 90°.

According to an aspect of the invention, the hollow part comprises a radial support extending radially from the hollow part and the first electromagnetic transducer is rigidly fixed to the radial support. The radial support mainly extends along the second axis which crosses the first axis.

Advantageously, an angle comprised between 0° and 45° can be formed between the second axis and a fourth axis which extends perpendicular to the first axis and which is included within the reference plane.

The tubular part and the radial support on which is mounted the first electromagnetic transducer can be formed as a single piece. Alternately, the tubular part and the radial support on which is mounted the first electromagnetic transducer are detachably attached to one another.

The invention further related to an end-effector assembly comprising at least one end-effector as described above and at least the guiding part equipped with the second electromagnetic transducer, the guiding part being received in the tubular part. The second electromagnetic transducer is attached in a fixed position on the guiding part. The guiding part and the tubular part comprise, at least on part of their respective periphery, complementary shapes, and wherein the cooperation of the complementary shapes prevents rotation of the guiding part with respect to the tubular part. The cooperation of the complementary shapes advantageously forms a stop to the insertion of the guiding part in the tubular part. The end-effector assembly can further comprise latching means, respectively arranged on external surface of the tubular part and on an internal surface of the hollow part.

Finally, the invention also related to a computer-assisted surgery system comprising at least
a robotic arm extending from a base to a flange,
the end-effector assembly as described above attached to the flange of the robotic arm,
a localization system comprising, at least, the first electromagnetic transducer and the second electromagnetic transducer.

Further details and advantages of the invention will appear in the following description. Embodiments of the invention will be described with reference to the drawings, in which
- Figure 1 and 1b respectively illustrate a perspective view of a computer-assisted surgery system of the invention and a focused view of an end-effector assembly of such computer-assisted surgery system;
- Figure 2 illustrates, in a perspective view, an end-effector according to a first embodiment of the present invention adapted to be used with the computer-assisted surgery system illustrated on figure 1, the end-effector comprising, at least a hollow part and a tubular part received in said hollow part, the hollow part being illustrated in a partial cross-section view;
- Figure 3 is a focused view of a part of the end-effector wherein the tubular part cooperated with the hollow part;
- Figure 4 illustrates, schematically, a guiding part adapted to be received in the end-effector of the invention, the guiding part being configured to receive a surgical tool;
- Figures 5a to 5d illustrate, schematically, the steps to assemble an end-effector assembly of the invention comprising an end-effector as illustrated on figure 2 and a guiding part as illustrated on figure 4, the guiding part being received in the tubular part of said end-effector;
- Figure 6 illustrates, in a perspective view, the tubular part of the end-effector according to a variant of the first embodiment of the invention,
- Figure 7 illustrates, in a perspective view, the tubular part of the end-effector according to a second embodiment of the invention,
- Figures 8 and 9 illustrate, respectively, two different variants of the first embodiment of the tubular part.

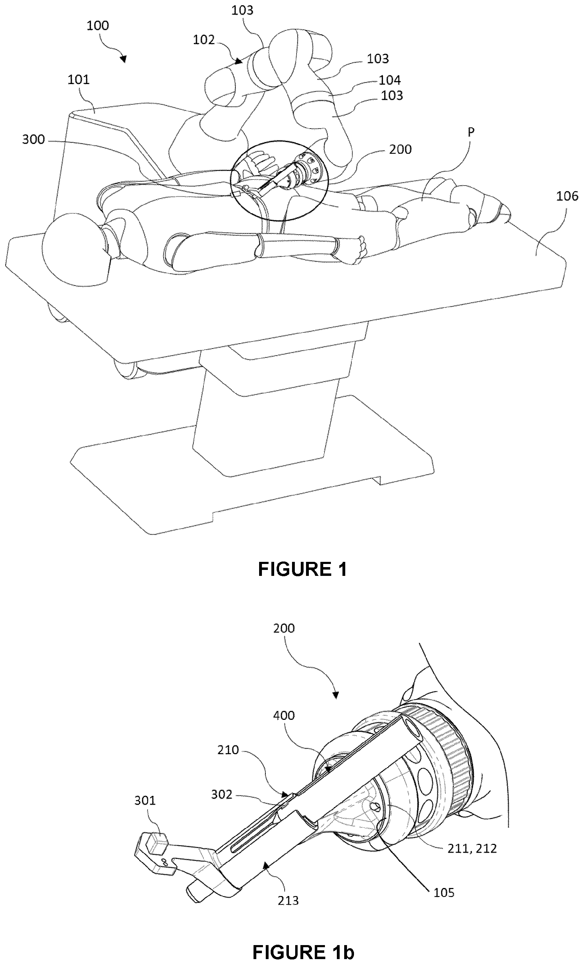

Figure 1 illustrates, schematically, a computer-assisted surgery system 100 - hereafter referred to as "the system 100" - according to the invention. Figure 1b is a focused view of an end-effector assembly 200 of said computer-assisted surgery system 100. The system 100 comprises a base 101 from which extends a robotic arm 102 itself comprising several links 103 linked to one another thanks to motorized joints 104. Those joints could alternatively be passive joints, i.e. deprived of motors, within the scope of the present invention. The robotic arm 102 could also be attached to a surgical table 106 instead of its base without departing from the scope of the invention.

The robotic arm 102 thus extends between the base 101 and a flange 105 to which the end-effector assembly 200 is releasably attached, as shown on figure 1b. This end-effector assembly 200 comprises an end-effector 210 described with more details below, and a guiding part 400 configured to receive a surgical tool itself adapted to be used to treat a region of interest of an anatomical structure of a patient P. Figure 1 illustrates a surgery which consists in implanting pedicular screws in the patient's spinal column. The illustrated guiding part 400 is thus configured to receive a screwdriver, but it could also be any instrument required to be operated at a specific depth from or in the patient anatomy. Obviously, this is only an example of the present invention and the system 100 could be used to perform other kind of spinal surgeries of even to treat other parts of the body, such as a knee, a hip of an ankle for instance. As detailed below, the surgical tool is rigidly and temporarily linked to the guiding part 400. The surgical tool can be any useful surgical tool, depending on the kind of treatment to be performed, such as a drill, a saw, a burr etc... within the scope of the invention, as long as it is configured to be inserted in the guiding part.

The system 100 finally comprises an electromagnetic localization system used to track poses of the end-effector assembly 200, and especially of the guiding part 400, and consequently of the surgical tool received in such guiding part 400 of the end-effector assembly, with respect to the region of interest. The electromagnetic localization system comprises at least one electromagnetic emitter adapted to emit at least one electromagnetic field, at least one electromagnetic receiver adapted to receive and measure the emitted electromagnetic field and at least one data processor- not illustrated here - adapted to receive and process the measurements of the electromagnetic field, so as to determine the current poses of the electromagnetic emitter(s) with respect to the electromagnetic receiver(s) and to determine, consequently, the current poses of the objects to which said emitter(s) / receiver(s) are attached. According to the illustrated embodiment, the electromagnetic emitter 300 is positioned on the back of the patient P, on his/her skin while the electromagnetic receivers 301, 302 are respectively fixed to a hollow part of the end-effector - such hollow part being described below - to the guiding part 400, and to the region of interest, here formed by several vertebrae of the patient P. The electromagnetic receiver(s) attached to the region of interest are not illustrated on figure 1, as the vertebrae of the patient P are not shown.

As it is well known in the art, the positions of the electromagnetic receiver(s) and of the electromagnetic emitter(s) could be swapped without modifying the results given by the electromagnetic localization system. Thus, the present specification refers indifferently to one or the other kind with the words "electromagnetic transducer(s)".

As known in the art, electromagnetic localization systems are sensitive to the presence of metallic materials which can be present in the surgical field and disturb the emitted electromagnetic field. As described below, the electromagnetic transducer attached to the end-effector is mobile so that the surgeon, or any other user of the system, can displace it to bring it closer to the electromagnetic transducer positioned on the patient, and especially on the back of the patient in the particular embodiment illustrated. Thus, as the electromagnetic transducers are arranged to be as close as possible to one another, the risk for the electromagnetic field to be disturbed between them decreases, consequently making the measurements more reliable.

Even if not illustrated, the system 100 could also be associated with an imaging device such as a C-arm for instance, without departing from the scope of the invention.

In the following specification, the words "top", "above", "below" and "bottom" are to be understood with their conventional means, when the computer-assisted surgery system is in use. Thus, the words "bottom" or "below" refers to positions closer to the patient that the positions referred to as "top" or "above" positions.

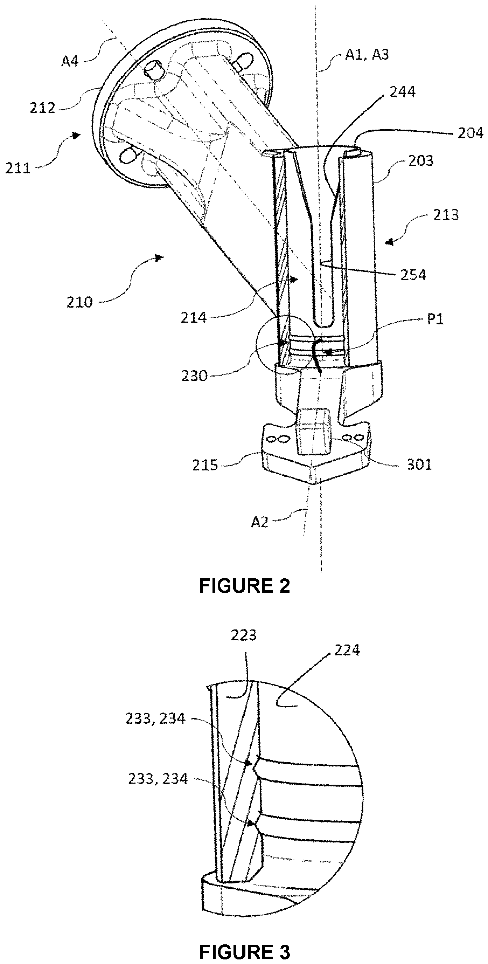

Now referring to figures 2 to 4d, we are going to describe the end-effector 210 and of the end-effector assembly 200 according to a first embodiment of the invention.

As illustrated on figure 2, the end-effector 210 according to the invention comprises at least a mounting part 211 adapted to be attached to the flange of the robotic arm. For this purpose, the mounting part 211 comprises an attachment plate 212 which can for instance be screwed to the flange of the robotic arm. Any other known attaching means can obviously replace this attachment plate 212 within the scope of the invention. The end-effector 210 then comprises a hollow part 213 rigidly attached to the mounting part 212. As shown, the hollow part 213 extends, mainly, along a first axis A1. According to the illustrated embodiment, the mounting part 212 and the hollow part 213 are made of a single piece, meaning that one cannot be detached from the other without damaging at least one of them. Obviously, this is only an example and the hollow part 213 could be rigidly attached to the mounting part by any other known means without departing from the scope of the invention.

The end-effector 210 further comprises a tubular part 214 received in the hollow part 213. On figure 2, the hollow part 213 is illustrated in a partial cross-sectional view, only to make the tubular part 214 visible. It is understood that in the final product, this hollow part 213 comprises a closed peripheral wall 203. Especially, as it will be described below, this closed peripheral wall 203 remains opened on its upper end and on its lower end opposed to one another along the first axis A1. As illustrated on the focused view of figure 3, which is an enlargement of the circled part of figure 2, latching means 230 are arranged on the tubular part 214 and on the hollow part 213 so that, once inserted in the hollow part, the tubular part 214 is fixed along a longitudinal direction, i.e. a direction parallel to the first axis A1, with respect to the hollow part 213. According to the particular example illustrated on figure 3, these latching means comprise at least one rib 234 formed on an external face 224 of the tubular part 214 and at least one groove 233 formed on an internal face 223 of the hollow part 213, the at least one rib 234 being received in the at least one groove 233. The tubular part 214 can thus be inserted into the hollow part 213 and remains maintained within such hollow part, thanks to the cooperation between the at least one rib 234 and the corresponding groove 233. According to the illustrated embodiment, two ribs are formed on the tubular part and two corresponding grooves are formed on the hollow part, but obviously, there could be more than two within the scope of the invention, so as to control and reduce manufacturing adjustments. As mentioned above, using ribs 234 and grooves 233 permits to lock the translational position of the tubular part with respect to the hollow part, while still allowing the rotation of said parts with respect to one another such that it can be inserted and removed by hand without special tools. The rib(s) 234 and the corresponding groove(s) 233 can extend on all the circumference of, respectively, the external face of the tubular part and the internal face of the hollow part, or only a portion of it, within the scope of the invention.

Advantageously, the latching means 230 are realized so as to provide interferential adjustment. For instance, the spacing of the ribs 234 arranged on the external face 223 of the hollow part 213 can be realized as 1/10^{th} larger or smaller than the corresponding spacing of the grooves 233 arranged on the internal surface 223 of the hollow part 213 so as to minimize assembly play and preserve device accuracy and concentricity

Referring back to figure 2, the tubular part 214 is rigidly attached to a first electromagnetic transducer 301. By "rigidly attached", we here mean that the displacement of one leads to the same displacement of the other. As described above, if the user/surgeon displaces the first electromagnetic transducer 301, for instance to improve the accuracy of the measurements, then the tubular part 214 is simultaneously rotated in the same direction and along the same angle. Especially, the tubular part 214 and the first electromagnetic transducer 301 are rigidly linked in rotation around the first axis A1.

According to the illustrated embodiment, the first electromagnetic transducer 301 is mounted on a radial support 215 which extends radially from the hollow part 213, and particularly from the peripheral wall 203 of said hollow part. According to the illustrated embodiment, the radial support 215 and the tubular part 214 are formed as a single piece, meaning that said radial support and said tubular part cannot be detached without damaging at least one of them. Alternatively, the radial support and the tubular part could be detachably attached within the scope of the invention. This support 215 mainly extends along a second axis A2 which crosses the first axis A1. The first axis A1 and the second axis A2 together define a reference plane P1. Advantageously, the second axis A2 can pass through a center of the first electromagnetic transducer 301.

According to the illustrated embodiment, the tubular part 214 extends along a third axis A3 which coincide with the first axis A1. Otherwise said, the first axis A1 also forms the axis of revolution of the tubular part 214. This tubular part 214 is mobile around the third axis A3, with respect to the hollow part 213 of the end-effector 210.

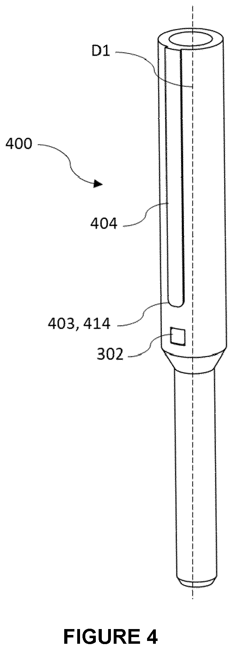

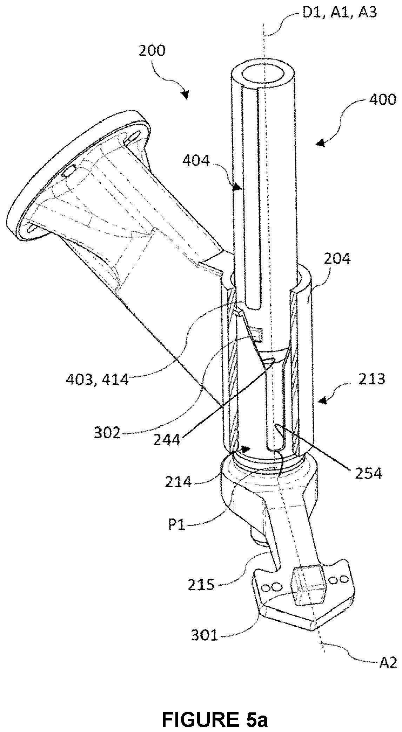

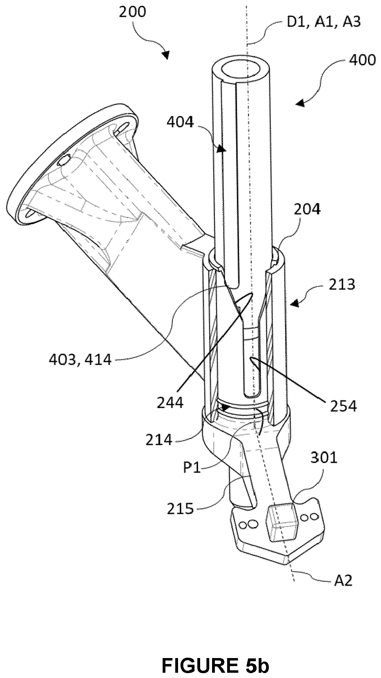

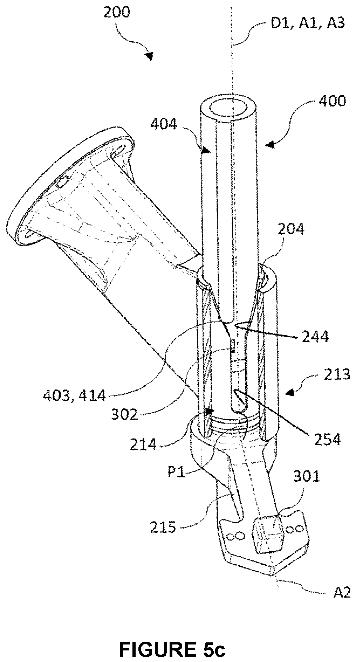

The tubular part 214 further comprises a first cooperative surface 244 formed on an insertion end 204 of the tubular part 214 and which is adapted to cooperate, in a keyed connection, with a second cooperative surface formed on the guiding part 400 and described below with reference to figure 4. This "insertion end" of the tubular part forms the side of said tubular part through which the guiding part is inserted to assemble the end-effector assembly. As it will be explained with more details below with reference to figures 5a to 5d, the first and the second cooperative surfaces are designed so that when the guiding part is inserted in the tubular part 214, parallel to the third axis A3, the longitudinal displacement created is transformed into a rotation to ensure a correct positioning of the guiding part with respect to the first electromagnetic transducer 301. Particularly, the guiding part is equipped with a second electromagnetic transducer 302 and the keyed connection is designed to ensure that once that the guiding part is inserted in the tubular part, the second electromagnetic transducer 302 lies, at least partially, in the reference plane P1, without any additional manipulation.

According to the illustrated embodiment, the first cooperative surface 244 can be formed as a helical surface. This helical surface can extend on all or part of the insertion end 204 of the tubular part 214. Advantageously, the helical surface extends on an angular sector greater than 90°.

The first cooperative surface 244 of the tubular part 214 is lengthened by a U-shaped notch 254 which forms a stop to the insertion of the guiding part and prevent it from rotating with respect to the tubular part 214. Advantageously, this U-shaped notch 254 can be formed so as to be at least partially included in the reference plane P1. This U-shaped notch 254 and its function will be described in further details below.

Figure 4 illustrates, schematically, the guiding part 400 adapted to be used to treat the concerned region of interest. The guiding part 400 mainly extends along a straight line D1 and presents a cylindrical shape. The guiding part 400 comprises the second cooperative surface 403 mentioned above and a longitudinal U-shaped rib 404 extending parallel to the straight line D1. In the end-effector assembly 200, this longitudinal U-shaped rib 404 is received in the U-shaped notch formed on the tubular part. The U-shaped rib 404 and the U-shaped notch thus form complementary shapes adapted to cooperate with one another to prevent the rotation of the surgical tool with respect to the tubular part. Obviously, the cooperation of the U-shaped rib with the U-shaped notch is only an example of two complementary shapes which can be used, but other complementary shapes could be implemented without departing from the scope of the invention as long as their cooperation prevents the surgical tool to rotate with respect to the tubular part. According to the illustrated example, the second cooperative surface 403 is formed as a bottom 414 of the U-shaped rib 404. Therefore, the tubular part 214 together with the hollow part 213 of the end-effector and with the guiding part 400 form a surgical guide configured to guide the surgical tool when it is inserted in said guiding part 400.

To permit its tracking by the localization system, the guiding part 400 is also equipped with the second electromagnetic transducer 302. As illustrated, this second electromagnetic transducer 302 is positioned below the U-shaped rib 404. This second electromagnetic transducer 302 is attached to the guiding part 400 so that its position remains fixed with respect to said guiding part 400. As the surgical tool is configured to be rigidly attached to the guiding part, the second electromagnetic transducer 302 permits to determine the current pose of the surgical tool inserted in said guiding part. Advantageously, the guiding part 400 comprises an abutment preventing the surgical tool to go deeper than needed for the planned surgery. Any known fixation device between the second electromagnetic transducer 302 and the guiding part 400, whether temporary or permanent, can be used within the scope of the invention. For instance, the second electromagnetic transducer 302 can be glued to the guiding part, or they can cooperate in a snap-fit connection or be semi-permanently assembled via screws and threaded holes.

Also, any known fixation device can be used between the surgical tool and the guiding part, as long as such fixation means permits a temporary attachment. The surgical tool can be assembled in the guiding part without residual motion using a variety of conventional semi-permanent, reversible clamping techniques. Advantageously, any elongated surgical tool can thus be used with the present invention, without needing to adapt it for receiving the electromagnetic transducer, as such electromagnetic transducer is attached to the guiding part, itself configured to be attached to said surgical tool.

Now referring to figures 5a to 5d, we're going to describe the steps to assemble the guiding part 400 and the end-effector 210 to obtain the end-effector assembly 200 of the invention.

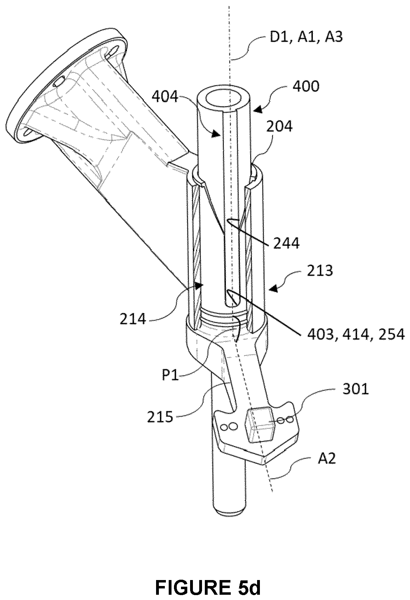

The guiding part 400 is first inserted in the tubular part 214 along the third axis A3, so that the straight line D1 coincide with the third axis A3. As the guiding part 400 is inserted, the second cooperative surface 403, here formed as the bottom 414 of the U-shaped rib 404, abuts the first cooperative surface 244 formed on said tubular part 214. As the user of the system keeps inserting the guiding part 400, such guiding part 400 is thus mechanically rotated, thanks to the cooperation between the first and the second cooperative surfaces, around the third axis A3, until a bottom of the U-shaped rib 404 abuts a bottom of the U-shaped notch 254, as shown on figure 5d. This cooperation thus allows a precise position control of the guiding part 400, and consequently of the surgical tool used to treat the anatomical region of interest. Therefore, once that the guiding part 400 is inserted in the tubular part, the U-shaped rib 404 lies, at least partially, in the reference plane P1. As the second electromagnetic transducer 302 is attached in a fixed position below such U-shaped rib 404, this second electromagnetic transducer 302 also lies, at least partially, within the reference plane P1. Obviously, this second electromagnetic transducer cannot be seen on figure 5d as it is hidden by the tubular part 214.

Optionally, the second electromagnetic transducer 302 could be attached at another location of the guiding part 400 as long as such other location permits to ensure that said second electromagnetic transducer 302 lies, at least partially, in the reference plane P1 once that guiding part 400 is completely inserted in the tubular part 214.

Furthermore, once that the guiding part 400 is inserted in the tubular part 214, sides of the U-shaped rib 404 are in contact with sides of the U-shaped notch 254, thus preventing any rotation of the guiding part 400 with respect to the tubular part 214. The guiding part 400 and the tubular part 214 are thus linked together, in rotation. Consequently, a rotation, around the first axis A1, of the first electromagnetic transducer 301 leads to a matched rotation of the guiding part 400, and especially of the second electromagnetic transducer 302 attached to such guiding part 400. This is particularly advantageous as when the first electromagnetic transducer is - as described above - rotated to be brought closer to the electromagnetic transducer arranged on the patient, the second electromagnetic transducer 302 is simultaneously brought closer to said electromagnetic transducer arranged on the patient. The whole end-effector assembly 200 thus permits to maintain the smallest distance possible between all three electromagnetic transducers without requiring separate adjustments, thus limiting the adverse effects of metallic material which can be present within the operating field, on the emitted electromagnetic field(s).

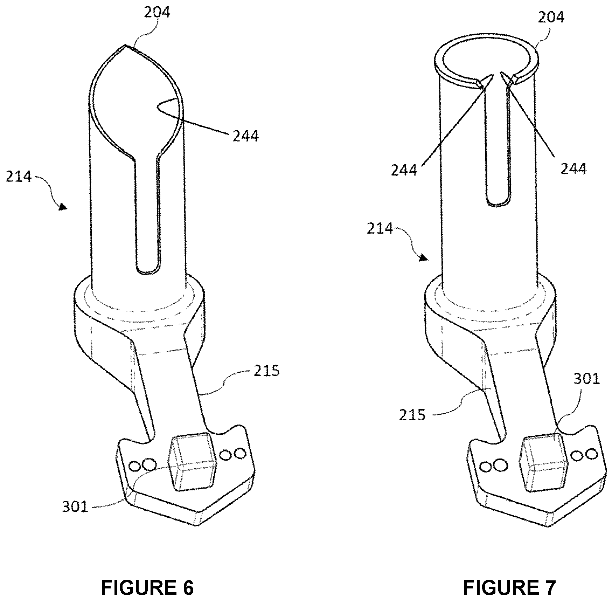

Figure 6 illustrates, in a perspective view, the tubular part 214 according to a variant of the first embodiment described above with reference to figure 2. As mentioned, the first cooperative surface 244 according to the first embodiment is formed as a helical surface which can extends on all or part of the insertion end 204 of the tubular part 214. Figure 6 illustrates a variant of the embodiment illustrated on figure 2 wherein the first cooperative surface 244 is formed as a helical surface which extends on the entirety of the insertion end 204 of the tubular part. This variant advantageously ensures that the guiding part will be guided to its final position wherein the complementary shapes formed on said guiding part and said tubular part cooperates, regardless of its position while it is inserted into said tubular part.

Figure 7 illustrates a second embodiment of the tubular part. This second embodiment differs from the first embodiment described above, in the shape of the first cooperative surface 244. As shown, the first cooperative surface 244 of the tubular part 214 according to the second embodiment of the invention comprises two chamfers, arranged on both sides of the U-shaped notch. This second embodiment thus still permits to guide the insertion of the guiding part to its final position, provided that the user of the system inserts it so that it can engage with the second cooperative surface. According to this second embodiment, the insertion end 204 of the tubular part 214 is flattened so that it can abut an insertion end of the hollow part, thus permitting to omit to form ribs on the external face 224 of the tubular part 214.

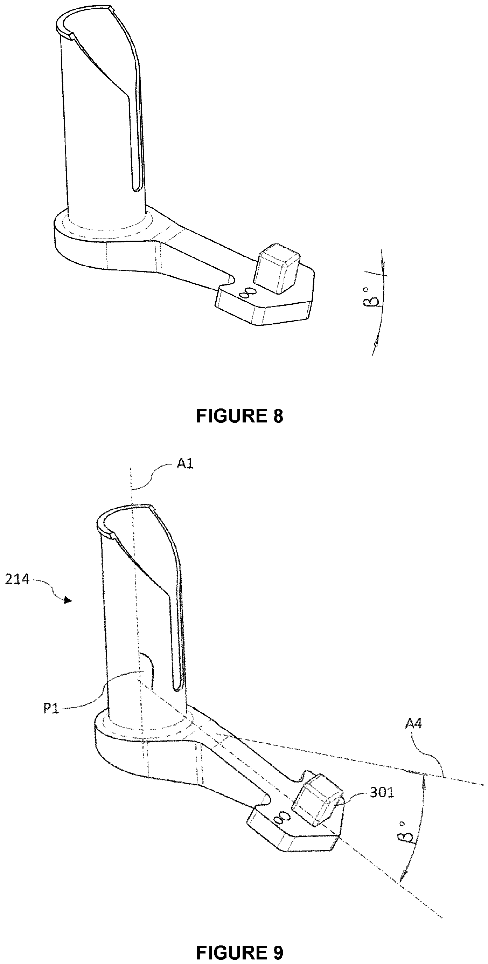

Finally, figures 8 and 9 illustrate two variants of the first embodiment of the tubular part 214, which can also be used as variant of the second embodiment to compensate the angular configuration of the robot with respect to the patient anatomy and the emitter location. The variants contribute to shortening the distance with the emitter and transducer 301.

To prevent the robotic arm to be cumbersome around the region of interest while ensuring that the electromagnetic transducer 301 attached to the end-effector is as close as possible to the electromagnetic transducer attached to the region of interest and while considering that the screw trajectory is seldom along a purely vertical direction, an angle β formed between the first axis A1 and a fourth axis A4 which extends perpendicular to the first axis A1 and which is included in the reference plane P1 is comprised between 0° and 45°. Especially, figure 8 illustrates a first variant wherein the angle β is equal to 15° ±5° and figure 9 illustrates a second variant wherein the angle β is equal to 30°±5°. Said angle β is advantageously chosen by considering the average screw trajectory angle with respect to the patient position.

## Claims

1. End-effector (210) for a robotic arm (102) adapted to be used with a surgical tool to treat a region of interest of an anatomical structure, the end-effector (210) comprising:
a. a mounting part (211) adapted to be attached to a flange (105) of the robotic arm (102),
b. a hollow part (213) extending mainly along a first axis (A1), the hollow part (213) being rigidly attached to the mounting part (211),
c. a first electromagnetic transducer (301) attached to the hollow part (213), the first electromagnetic transducer (301) being mobile around the first axis (A1), the first axis (A1) and a second axis (A2) passing through the first electromagnetic transducer (301) and crossing the first axis (A1) defining a reference plane (P1),
d. a tubular part (214) received in the hollow part (213), the tubular part (214) being in a fixed position with respect to the first electromagnetic transducer (301),
e. a guiding part (400) configured to be received in the tubular part (214) and configured to receive the surgical tool, the guiding part (400) being equipped with a second electromagnetic transducer (302),
wherein the tubular part (214) comprises a first cooperative surface (244) adapted to cooperate, in a keyed connection, with a second cooperative surface (403) formed on the guiding part (400) and wherein the keyed connection is adapted to ensure that at least part of the second electromagnetic transducer (302) lies in the reference plane (P1).

2. End-effector (210) according to the preceding claim, wherein the tubular part (214) extends, mainly, along a third axis (A3), and wherein the first cooperative surface (244) is formed so that a translation of the second cooperative surface (403) along the third axis (A3) and against the first cooperative surface (244) is transformed into a rotation of the second cooperative surface (403) with respect to the first cooperative surface (244), around the third axis (A3).

3. End-effector (210) according to any of the preceding claims, wherein the first cooperative surface (244) comprises at least one chamfer.

4. End-effector (210) according to any of claims 1 or 2, wherein the first cooperative surface (244) is formed as a helical surface.

5. End-effector (210) according to the preceding claim, wherein the helical surface is formed on an angular sector of an insertion end (204) of the tubular part (214) greater than 90°.

6. End-effector (210) according to any of the preceding claims, wherein the hollow part (213) comprises a radial support (215) extending radially from the hollow part (213) and wherein the first electromagnetic transducer (301) is rigidly fixed to the radial support (215).

7. End-effector (210) according to the preceding claim, wherein the radial support (215) mainly extends along the second axis (A2) which crosses the first axis (A1).

8. End-effector (210) according to any of the preceding claims, wherein an angle (β) comprised between 0° and 45° is formed between the second axis (A2) and a fourth axis (A4) which extends perpendicular to the first axis (A1) and which is included within the reference plane (P1).

9. End-effector (210) according to any of the preceding claims in combination with any of claims 6 or 7, wherein the tubular part (214) and the radial support (215) on which is mounted the first electromagnetic transducer (301) are formed as a single piece.

10. End-effector (210) according to any of claims 1 to 8 in combination with any of claims 6 or 7, wherein the tubular part (214) and the radial support (215) on which is mounted the first electromagnetic transducer (301) are detachably attached to one another.

11. End-effector assembly (200) comprising at least one end-effector (210) according to any of the preceding claims and at least the guiding part (400) equipped with the second electromagnetic transducer (302), the guiding part (400) being received in the tubular part (214).

12. End-effector assembly (200) according to the preceding claim, wherein the second electromagnetic transducer (302) is attached in a fixed position on the guiding part (400).

13. End-effector assembly (200) according to any of claims 11 or 12, wherein the guiding part (400) and the tubular part (214) comprise, at least on part of their respective periphery, complementary shapes, and wherein the cooperation of the complementary shapes prevents rotation of the guiding part (400) with respect to the tubular part (214).

14. End-effector assembly (200) according to any of claims 11 to 13, wherein the cooperation of the complementary shapes forms a stop to the insertion of the guiding part (400) in the tubular part (214).

15. End-effector assembly (200) according to any of claims 11 to 14, comprising latching means (230), respectively arranged on external surface (224) of the tubular part (214) and on an internal surface (223) of the hollow part (213).

16. Computer-assisted surgery system (100) comprising at least
a. a robotic arm (102) extending from a base (101) to a flange (105),
b. the end-effector assembly (200) according to any of claims 11 to 15 attached to the flange (105) of the robotic arm (102),
c. a localization system comprising, at least, the first electromagnetic transducer (301) and the second electromagnetic transducer (302).
